# EUROPEAN PATENT APPLICATION

(11) **EP 3 184 111 A1**
(43) Date of publication of application: **28.06.2017**
(21) Application number: 15382651.6
(22) Date of filing: 21.12.2015
(51) Int. Cl.: A61K 31/70, A61K 31/7016, A61K 31/702, A61K 31/715, A61K 31/716, A61K 31/718, A23L 2/52, A23L 2/66

(54) **COMPOSITIONS COMPRISING CARBOHYDRATES**

(71) Applicant: Abbott Laboratories, Abbott Park, IL 60064 (US)
(72) Inventor: LÓPEZ PEDROSA, José Maria, 18004 GRANADA (ES); MANZANO MARTÍN, Manuel Cristóbal, 18004 GRANADA (ES); MARTIN MARTIN, Maria Jesús, 18004 GRANADA (ES); RUEDA CABRERA, Ricardo, 18004 GRANADA (ES)
(74) Representative: Boult Wade Tennant

(57) **Abstract**

The present invention relates to methods comprising administering to a subject a composition comprising at least one slow rate of digestion simple carbohydrate, at least one complex carbohydrate, and at least one further carbohydrate selected from a nonabsorbent carbohydrate and an indigestible carbohydrate. The methods of the invention relate to inducing satiety, increasing energy expenditure and/or preventing or treating obesity in the subject.

## Description

### FIELD OF THE INVENTION

The present invention is directed to methods based upon administration of compositions or nutritional compositions comprising a specific carbohydrate system. In particular, such compositions comprise at least one slow rate of digestion simple carbohydrate, at least one complex carbohydrate, and at least one further carbohydrate selected from a nonabsorbent carbohydrate and an indigestible carbohydrate. Methods are described for inducing satiety, for increasing energy expenditure and for preventing and/or treating obesity in a subject.

### BACKGROUND

The prevalence of obesity in adolescents and adults has increased rapidly over the past 20 years in the United States and globally, and continues to rise. Obesity is a condition classically defined based on the percentage of body fat or, more recently, the body mass index or BMI of an individual. BMI is defined as the ratio of weight in kilograms divided by the height in meters squared.

The relative risk of developing conditions such as insulin resistance, diabetes, dyslipidemia, non-alcoholic fatty liver, hypertension and coronary heart disease is greatly increased in obese people. As such, obesity is a serious public health problem that needs to be addressed.

Although a human's risk of becoming obese is determined in part by genetic factors, most obese individuals display an imbalance in energy intake and energy expenditure wherein the former exceeds the latter. For example, if a high energy intake is accompanied by a sedentary lifestyle, any excess energy ingested is converted into fat storages promoting an increase in body weight. Given the magnitude of the obesity problem in the global population, there is a need to develop improved methods and compositions for regulating energy balance and consequently body weight. The present invention seeks to address this issue.

### SUMMARY OF INVENTION

The present inventors have found that a composition comprising a specific carbohydrate system or carbohydrate blend can be used to regulate energy balance by affecting both energy intake and energy expenditure.

In a first aspect, the present invention provides a method of inducing satiety in a subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate.

In a second aspect, the invention provides a method of increasing energy expenditure in a subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate.

In a third aspect, the invention provides a method of preventing or treating obesity in a subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate.

In all aspects of the invention, in certain embodiments, the subject to which the composition is administered is a human subject, optionally a human subject having a healthy bodyweight or optionally a human subject classified as obese. In certain embodiments of the methods described herein, the subject is not a pregnant woman or a lactating woman.

In all aspects of the invention, in certain embodiments, the composition comprises at least one slow rate of digestion simple carbohydrate selected from the group consisting of isomaltulose and sucromalt and/or at least one complex carbohydrate selected from the group consisting of a maltodextrin, corn starch, rice starch and wheat starch and/or at least one nonabsorbent carbohydrate selected from the group consisting of inulin, dietary insoluble fibers, and digestion-resistant maltodextrins and/or at least one indigestible carbohydrate selected from the group consisting of fructooligosaccharides, galactooligosaccharides, transgalactooligosaccharides and xylooligosaccharides.

The invention will be further understood with reference to the following numbered clauses:
1. A method of inducing satiety in a subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate.
2. The method of clause 1 wherein satiety is induced such that the subject's energy intake is reduced.
3. A method of increasing energy expenditure in subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate.
4. A method of preventing or treating obesity in a subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate.
5. The method of clause 4, wherein the composition administered to the subject induces satiety and/or increases energy expenditure in the subject.
6. The method of any of clauses 1-5 wherein the subject is a human.
7. The method of clause 6 wherein the subject is selected from a child, a toddler, an adolescent, an adult or an elderly adult.
8. The method of clause 6 or clause 7 wherein the subject is not a pregnant woman or a lactating woman.
9. The method of any of clauses 1-8 wherein the subject has a healthy bodyweight.
10. The method of any of clauses 1-8 wherein the subject is overweight or obese.
11. The method of any of clauses 1-10 wherein the composition comprises at least one slow rate of digestion simple carbohydrate, at least one complex carbohydrate, at least one nonabsorbent carbohydrate and at least one indigestible carbohydrate.
12. The method of any of clauses 1-11 wherein the slow rate of digestion simple carbohydrate is a carbohydrate comprised of monosaccharide or disaccharide sugars and that is low glycemic and/or low insulinemic such that it provides a relatively low rise in blood glucose over time.
13. The method of any of clauses 1-12 wherein the slow rate of digestion simple carbohydrate is selected from the group consisting of isomaltulose and sucromalt.
14. The method of any of clauses 1-13 wherein the complex carbohydrate is a carbohydrate comprising a chain of three or more monosaccharides.
15. The method of any of clauses 1-14 wherein the complex carbohydrate is selected from the group consisting of a maltodextrin, corn starch, rice starch and wheat starch.
16. The method of any of clauses 1-14 wherein the complex carbohydrate comprises maltodextrin having a Dextrose Equivalent of from 3 to 25.
17. The method of any of clauses 1-14 wherein the complex carbohydrate comprises maltodextrin having a Dextrose Equivalent of from 9 to 16.
18. The method of any of clauses 1-17 wherein the nonabsorbent carbohydrate is a carbohydrate that is not absorbed in the upper intestinal tract.
19. The method of any of clauses 1-18 wherein the nonabsorbent carbohydrate is selected from the group consisting of inulin, dietary insoluble fibers, and digestion-resistant maltodextrins.
20. The method of any of clauses 1-19 wherein the indigestible carbohydrate is selected from the group consisting of fructooligosaccharides, galactooligosaccharides, trans-galactooligosaccharides and xylooligosaccharides.
21. The method of any of clauses 1-20 wherein the composition comprises a slow rate of digestion simple carbohydrate that is isomaltulose, a complex carbohydrate that is maltodextrin having a DE of 9 to 16, a nonabsorbent carbohydrate that is Fibersol 2E and an indigestible carbohydrate that is fructooligosaccharide.
22. The method of any of clauses 1-21 wherein the composition comprises from 40% to 80% by weight slow rate of digestion simple carbohydrate, from 1% to 15% by weight complex carbohydrate, from about 5% to about 25% by weight nonabsorbent carbohydrate, and from 1% to 20% by weight indigestible carbohydrate.
23. The method of any of clauses 1-21 wherein the composition comprises from 60% to 70% by weight slow rate of digestion simple carbohydrate, from 6% to 10% by weight complex carbohydrate, from 5% to 20% by weight nonabsorbent carbohydrate, and from 2% to 15% by weight indigestible carbohydrate.
24. The method of any of clauses 1-23 wherein the subject is administered with from 20 to 175 grams of the carbohydrate system per day.
25. The method of any of clauses 1-23 wherein the subject is administered with from 90 to 125 grams of the carbohydrate system per day.
26. The method of any of clauses 1-25 wherein the composition is a nutritional composition.
27. The method of clause 26 wherein the nutritional composition comprises one or more additional macronutrients, optionally selected from protein, lipid and/or additional carbohydrate.

### BRIEF DESCRIPTION OF DRAWINGS

**Figure 1** shows the effects of experimental diets on body weight and food intake of rats after 90 days of nutritional treatment. **A)** shows the increase in body weight over the time course of the study; **B)** shows the total body weight gain over the 90 day period; **C)** shows the differences in food intake between the different experimental groups.
Figure 2 shows changes in the body composition of rats after 90 days of feeding with experimental diets. A) shows the percentage of fat mass; B) shows the percentage of lean mass.
Figure 3 shows the effects of experimental diets on oxygen consumption (VO2) and energy expenditure (EE) of rats during 24 h after 60 days of feeding with the experimental diets. A) Oxygen consumption; B) Energy expenditure.

### DETAILED DESCRIPTION

### A. Definitions

Unless defined otherwise, all technical and scientific terms used herein have the same meaning as commonly understood by the ordinary person skilled in the art to which the invention pertains. Without limiting any term, further clarifications of some of the terms used herein are provided below.

The terms "nutritional formula" or "nutritional product" or "nutritional composition," as used herein, are used interchangeably and, unless otherwise specified, refer to nutritional liquids, nutritional solids, nutritional semi-liquids, nutritional semi-solids, nutritional powders, nutritional supplements, and any other nutritional food product as known in the art. The nutritional powders may be reconstituted to form a nutritional liquid, all of which comprise one or more of fat, protein and carbohydrate, and are suitable for oral consumption by a human.

The term "nutritional liquid," as used herein, unless otherwise specified, refers to nutritional products in ready-to-drink liquid form, concentrated form, and nutritional liquids made by reconstituting the nutritional powders described herein prior to use.

The term "nutritional powder," as used herein, unless otherwise specified, refers to nutritional products in flowable or scoopable form that can be reconstituted with water or another aqueous liquid prior to consumption and includes both spray dried and dry-mixed/dry-blended powders.

The term "nutritional semi-solid," as used herein, unless otherwise specified, refers to nutritional products that are intermediate in properties, such as rigidity, between solids and liquids. Some semi-solids examples include puddings, gelatins, and doughs.

The term "nutritional semi-liquid," as used herein, unless otherwise specified, refers to nutritional products that are intermediate in properties, such as flow properties, between liquids and solids. Some semi-liquids examples include thick shakes and liquid gels.

The terms "retort" and "retort sterilized" are used interchangeably herein, and unless otherwise specified, refer to the common practice of filling a container, most typically a metal can or other similar package, with a nutritional liquid and then subjecting the liquid-filled package to the necessary heat sterilization step, to form a retort sterilized nutritional liquid product.

The terms "aseptic" and "aseptic sterilized" are used interchangeably herein, and unless otherwise specified, refer to the manufacture of a packaged product without reliance upon the above-described retort packaging step, wherein the nutritional liquid and package are sterilized separately prior to filling, and then are combined under sterilized or aseptic processing conditions to form a sterilized, aseptically packaged, nutritional liquid product.

The terms "administer," "administering," "administered," and "administration," as used herein, unless otherwise specified, should be understood to include providing a nutritional composition to a subject, the act of consuming a nutritional composition (self-administration), and combinations thereof. In addition, it should be understood that the methods disclosed herein may be practised with or without doctor supervision or other medical direction.

The term "subject" as used herein, unless otherwise specified, refers to a mammal, including companion animals, livestock, laboratory animals, working animals, sport animals, and humans. In preferred embodiments, the subject is a human.

All percentages, parts and ratios as used herein, are by weight of the total composition, unless otherwise specified. All such weights, as they pertain to listed ingredients, are based on the active level and, therefore, do not include solvents or by-products that may be included in commercially available materials, unless otherwise specified.

Numerical ranges as used herein are intended to include every number and subset of numbers within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

All references to singular characteristics or limitations of the present disclosure shall include the corresponding plural characteristic or limitation, and vice versa, unless otherwise specified or clearly implied to the contrary by the context in which the reference is made. Unless otherwise specified, "a," "an," "the," and "at least one" are used interchangeably. Furthermore, as used in the description and the appended claims, the singular forms "a," "an," and "the" are inclusive of their plural forms, unless the context clearly indicates otherwise.

All combinations of method or process steps as used herein can be performed in any order, unless otherwise specified or clearly implied to the contrary by the context in which the referenced combination is made.

### B. Methods of the invention

### Methods of inducing satiety

In a first aspect, the present invention is directed to methods of inducing satiety in a subject. The methods involve administering a composition comprising a specific carbohydrate system to the subject wherein the specific carbohydrate system is as defined in the embodiments described elsewhere herein. The present invention also encompasses a composition for use in a method of inducing satiety in a subject wherein the composition comprises the specific carbohydrate system according to the embodiments described herein.

The term "satiety" means the condition or sensation of feeling full after eating. The sensation of satiety may last for a significant period, for example a period of a few hours, before the sensation of hunger returns. Satiation is the condition or sensation of feeling full during eating and is caused by the cumulative effect of inhibitory signals from sensory, cognitive, digestive and hormonal origins induced by the ingestion of food substances. The sensation of satiation usually develops in a subject as a period of feeding progresses and is a biological mechanism intended to bring feeding to an end.

The methods described herein aim to induce satiety by means of a composition comprising a specific combination of different types of carbohydrate. It has been found that this specific carbohydrate system is able to reduce food intake in an experimental system and has therefore been implicated in satiety control.

The term "inducing satiety" as used herein is intended to mean satiety induced to a level wherein the subject's energy intake or food intake is reduced. This reduction may be determined by comparing the subject's energy/food intake with a subject provided with an isocaloric diet, but without the composition comprising the specific carbohydrate system as described herein. By "isocaloric diet" is meant a diet having an equivalent number of calories provided by corresponding quantities of protein, fat, carbohydrate etc. Alternatively, this reduction may be determined by assessing the subject's energy/food intake prior to administration of the composition and comparing this with the subject's energy/food intake during or after a period of administering the composition comprising the specific carbohydrate system described herein.

The compositions for administration according to the methods described herein induce satiety and thereby reduce overall energy intake i.e. the number of calories consumed is reduced. Energy intake may be reduced per serving or per meal or may be reduced over a defined period of administration of the compositions described herein. For example, satiety may be induced according to the methods described herein such that energy intake is reduced over a period of at least 8 hours, at least 24 hours, at least 7 days, at least 14 days, at least 30 days, at least two months, at least six months.

### Methods of increasing energy expenditure

In a second aspect, the present invention is directed to methods of increasing energy expenditure in a subject. The methods involve administering a composition comprising a specific carbohydrate system to the subject wherein the specific carbohydrate system is as defined in the embodiments described elsewhere herein. The present invention also encompasses a composition for use in a method of increasing energy expenditure in a subject wherein the composition comprises the specific carbohydrate system according to the embodiments described herein.

The term "energy expenditure" means the amount of energy used by an individual in any given period. A person's energy expenditure is the sum of the following: basal metabolism; the thermic effect of food; thermoregulation (control of body temperature); and energy expended through physical activity.

The methods of the present invention involve administering a composition comprising a specific carbohydrate system such that energy expenditure is increased. This increase may be determined by comparing the energy expenditure of an individual prior to and after administration of the composition, preferably wherein all other aspects of dietary intake are equivalent at the time of determining energy expenditure. The increase in energy expenditure may be determined by measuring the energy expenditure of an individual at a first time point prior to administration of the composition and calculating energy expenditure at one or more defined time points after a period during which the subject is administered the composition a number of times. For example, an individual may consume a serving of the composition at least once daily, at least twice daily, at least three times daily for a period of at least 7 days, at least 14 days, at least one month, at least two months and the increase in energy expenditure may be determined at the start and end of the time period.

In certain embodiments, the increase in energy expenditure may shift the energy balance in the subject administered the composition such that energy expenditure exceeds energy intake. In such embodiments, the methods are for increasing energy expenditure in a subject such that the subject experiences a loss in body weight over a period of time, for example at least 7 days, at least 14 days, at least 30 days, at least two months, at least six months. In such embodiments, the methods of increasing energy expenditure may be regarded as methods of treating obesity.

The energy expenditure of an individual can be determined using standard techniques known in the art. These include, but are not limited to, indirect and direct calometric and non-calometric methods as described, for example, in the paper by Levine (Measurement of energy expenditure, Public Health Nutr. 2005: 8(7A):1123-32), the contents of which are incorporated herein in their entirety.

In all of the methods described herein, the composition may be formulated for administration to the subject according to any suitable route of administration. In certain embodiments, the composition is formulated for oral administration, including a nutritional composition formulated for oral administration. As used herein "oral administration" and "administered orally" refer to any form of administration in which the composition is introduced into the subject's digestive system, including the stomach and small intestine. For example, oral administration includes nasogastric intubation, in which a tube is run through the nose to the stomach of the subject to administer food or drugs. Any solid, liquid, semi-solid, semi-liquid, or powder product form, including combinations or variations thereof, are suitable for use in the exemplary embodiments described herein, provided that such forms allow for safe and effective oral delivery to the subject via oral consumption of the ingredients as also defined herein.

The methods described herein may involve administering a composition comprising a specific carbohydrate system to a subject as part of a regular meal or to replace a regular meal. For example, the composition may serve as a supplemental nutritional source or the sole source of nutrition for the subject. Wherein the composition is administered as the sole source of nutrition, this may be the sole source of nutrition for a single meal or may be the sole source of nutrition replacing the subject's normal nutritional intake over a defined period, for example, a period of at least 8 hours, a period of at least 24 hours, a period of at least 7 days.

For embodiments wherein the composition is administered as part of a regular meal, the composition may be administered, for example consumed, prior to or during consumption of the meal. In embodiments wherein the composition is administered to replace a regular meal or is intended to serve as the sole source of nutrition for the subject, the composition may be formulated as a nutritional product intended to provide a certain amount of energy per serving. For example, the nutritional composition may be formulated so as to provide up to 500 kcal of energy per serving, including from 20 kcal to 500 kcal, from 75 kcal to 500 kcal, from 150 kcal to 500 kcal, from 200 kcal to 500 kcal, from 300 kcal to 500 kcal, or from 400 kcal to 500 kcal per serving.

In the methods described herein, compositions comprising the specific carbohydrate system may be administered to any suitable subject, preferably wherein the subject is human. The human subject may be selected from an infant, a toddler, a child, an adolescent, an adult or an elderly adult. As used herein, an "infant" refers to a human having an age of up to 12 months, a "toddler" refers to a human having an age of greater than one year up to three years of age, a "child" refers to a human having an age of greater than three years up to twelve years of age, an "adolescent" refers to a human having an age of 12 years to 18 years, an "adult" refers to a human having an age of 18 years to 50 years, and an "elderly adult" refers to a human having an age of greater than 50 years.

In certain embodiments, the compositions are administered to a healthy subject or a subject having a healthy bodyweight i.e. a subject who is not classed as obese. Healthy bodyweight can be calculated based on a subject's height, gender and age. A subject having a healthy bodyweight may also be identified based on body mass index or "BMI" calculations, which take into account height and weight. The World Health Organization's recommended body weight based on BMI values is shown in Table 1 below.

**Table 1 WHO's recommended BMI values for adults**

| **Category** | **BMI range - kg/m²** |
|---|---|
| Severe thinness | <16 |
| Moderate thinness | 16-17 |
| Mild thinness | 17-18.5 |
| Normal | 18.5-25 |
| Overweight | 25-30 |
| Obese Class I | 30-35 |
| Obese Class II | 35-40 |
| Obese Class III | >40 |

In certain embodiments, the methods as described herein involve administering a composition to an adult subject having a BMI of 25 or less, a BMI of 18.5-25 or a BMI of 18.5-24.9. Wherein the methods involve administration of a composition comprising the specific carbohydrate system described herein to a healthy subject, the method may be carried out for the purposes of regulating energy balance in the subject, for example to ensure that energy intake and energy expenditure are as closely matched as possible. In such methods, the aim may be for the subject to maintain a healthy bodyweight, for example as part of healthy lifestyle. Such methods may be regarded as non-therapeutic.

In certain embodiments, the compositions are administered to a subject classed as overweight or obese, for example a subject having a BMI of 25 or greater, or a BMI of 30 or greater. In such embodiments, the method may be regarded as a method of treating overweight or obesity on the basis that the induction of satiety and/or the increase in energy expenditure is intended to shift the subject's energy balance to a position wherein energy expenditure exceeds energy intake such that there is a reduction in body weight of the subject.

### Methods of preventing or treating obesity

In a third aspect, the present invention provides a method of preventing or treating obesity in a subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate. The present invention also encompasses a composition for use in a method of preventing or treating obesity in a subject wherein the composition comprises the specific carbohydrate system according to the embodiments described herein. As used herein, preventing or treating obesity in a subject is intended to encompass the prevention or treatment of "overweight" in a subject.

The term "obesity" is used to describe individuals having an excess of body fat, typically an excess of body fat such that there is a negative effect on the individual's health. The term "obesity" as used herein is intended to encompass the condition of "overweight". Individuals who are classified as overweight or obese have accumulated excess body fat as a result of an imbalance between energy intake and energy expenditure. Although genetic factors influence the development of overweight or obesity, in many people overweight or obesity is caused by excessive food intake typically accompanied by a sedentary lifestyle.

There are various methods of determining whether an individual is overweight or obese. Body mass index (or BMI) is the most common method of measuring overweight or obesity and as noted above, according to the World Health Organisation's guidance in this area, an overweight adult is one having a BMI of 25-30 and an obese adult is one having a BMI of 30 or more (see Table 1).

The present invention is directed to methods of preventing obesity in an adult subject and in certain embodiments, such methods involve preventing the subject from developing a BMI of 25 or more, optionally 30 or more. In certain embodiments, the methods of preventing obesity described herein involve administering a composition comprising a specific carbohydrate system of the invention to an adult subject having a normal bodyweight or a BMI of between 18.5 and 25 or between 18.5 and 24.9, such that the subject does not increase in weight. In certain embodiments, the methods of preventing obesity involve administering a composition comprising a specific carbohydrate system of the invention to an adult subject classified or diagnosed as overweight, for example an adult subject having a BMI of 25-30 or a BMI of 25-29.9, such that the overweight subject does not increase further in weight so as to become obese.

In certain embodiments, the methods of preventing obesity described herein involve administering a composition to a subject wherein the subject is identified as "at risk" of becoming obese. Such subjects may include those at risk due to environmental factors and/or those identified as at risk due to genetic risk factors.

In certain embodiments, the methods of preventing obesity described herein involve administering a composition to a toddler, child or adolescent so as to prevent the toddler, child or adolescent from becoming obese. Obesity in children can also be measured by calculating BMI; however, age and sex appropriate growth references must be used to correctly determine weight status. Weight status in children (aged 2-18 years) can be defined using the UK90 clinical cut points as shown in Table 2 below.

**Table 2 UK90 clinical cut points for body weight in children**

| | |
|---|---|
| **Clinically very underweight** | **< 0.4th centile** |
| **Clinically low weight** | **< 2nd centile** |
| **Clinically healthy weight** | **>2 - < 91the centile** |
| **Clinically overweight** | **> 91st centile** |
| **Clinically obese** | **> 98th centile** |
| **Clinically extremely obese** | **> 99.6th centile** |

In certain embodiments, the methods of preventing obesity described herein involve administering a composition to a child or adolescent wherein the child or adolescent has a clinically healthy weight (>2 - < 91the centile). The methods of preventing obesity may involve administering a composition to a child or adolescent wherein the child or adolescent is clinically overweight (≥ 91^{st} centile - <98^{th} centile) such that the child/adolescent does not increase in weight so as to be obese.

Weight status in children may also be assessed using the WHO Child Growth Standards based on length/height, weight and age, as described in Acta Pædiatrica, 2006; Suppl 450: 76-85, the contents of which are incorporated herein in their entirety.

The present invention is also directed to methods of treating obesity in a subject. In certain embodiments, such methods involve treating an adult subject having a BMI of 25-30 (wherein the subject is overweight) or a BMI of 30 or more (wherein the subject is obese). In certain embodiments, such methods involve treating a child or adolescent in the 91^{st} centile or above (wherein the child/adolescent is overweight) or in the 98^{th} centile or above (wherein the child/adolescent is obese) according to the UK90 clinical cut system shown above.

In addition to BMI, obesity, particularly in adults, may be measured by alternative techniques. For example, obesity may be measured by a skin fold thickness test that measures subcutaneous fat located directly beneath the skin by grasping a fold of skin and subcutaneous fat and measuring it using calipers. It is used mainly to determine relative fatness and the percentage of body fat. Obesity or body fat can also be measured by determining a subject's waist circumference wherein for men, a measure of ≥ 94 cm is indicative of increased health problems and for women, a measure of ≥ 80 cm is indicative of increased health problems. Obesity can also be measured by bio-impedance, which measures the impedance or opposition to the flow of a very small electric current as it passes through the body. As lean mass is made up of 73% water and fat has no water content, this method estimates lean tissue mass (which acts as a conductor) and fat mass (which acts as an insulator), through changes in voltage. In certain embodiments, the methods of treating obesity as described herein may be carried out on subjects classed as "obese" based on any of these measures.

In the context of the present methods, the term "treating obesity" may mean the alleviation of symptoms displayed by an overweight or obese individual. In certain embodiments, "treating obesity" in a subject means achieving a stable body weight in an individual wherein body weight does not change or increase over a period of time. In certain embodiments, "treating obesity" in a subject means achieving a reduction in body weight in an overweight or obese individual. For example, the BMI of the subject may fall by at least two BMI units, by at least four BMI units and/or may fall such that the subject's BMI is less than 30 or less than 25. Alternatively or in addition, "treating obesity" may involve preventing or reducing the risk of developing co-morbidities associated with these conditions including but not limited to: insulin resistance; diabetes; dyslipidemia; non-alcoholic fatty liver; hypertension; and coronary heart disease.

Compositions containing the carbohydrate system as described herein have been previously described for preventing fat accumulation in women during gestation, see in particular European patent publication no. EP2777404A1. Pregnant women, especially obese pregnant women, can develop an insulin-resistant state such that excess fat is accumulated during the gestational period. This gestational-related fat is predominantly accumulated centrally and represents a combination of truncal fat and visceral fat. Compositions containing the specific carbohydrate system as described herein have been described as a means to prevent excessive accumulation of gestational-related fat.

As exemplified herein, the present inventors have found that compositions comprising the specific carbohydrate system described herein can induce satiety and increase energy expenditure. This observation has opened up the possibility of using the compositions described herein to prevent or treat obesity in any subject, including any human subject. In certain embodiments, the methods for preventing and/or treating obesity as described herein involve administering a composition to a human subject wherein the subject is not a pregnant woman and/or is not a lactating woman. In certain embodiments, the methods for preventing and/or treating obesity as described herein are not for preventing fat accumulation in women during gestation.

In certain embodiments, the methods as described herein are for preventing or treating obesity by inducing satiety and/or by increasing energy expenditure in a subject. The terms "inducing satiety" and "increasing energy expenditure" have the same meaning as described in the first and second aspects of the invention discussed above.

In the methods of preventing and/or treating obesity described herein, the composition may be formulated for administration to the subject according to any suitable route of administration. In certain embodiments, the composition is formulated for oral administration, including a nutritional composition formulated for oral administration. As used herein "oral administration" and "administered orally" refer to any form of administration in which the nutritional composition is introduced into the subject's digestive system, including the stomach and small intestine. For example, oral administration includes nasogastric intubation, in which a tube is run through the nose to the stomach of the subject to administer food or drugs. Any solid, liquid, semi-solid, semi-liquid, or powder product form, including combinations or variations thereof, are suitable for use in the exemplary embodiments described herein, provided that such forms allow for safe and effective oral delivery to the subject via oral consumption of the ingredients as also defined herein.

The methods described herein may involve administering a composition comprising a specific carbohydrate system to a subject as part of a regular meal or to replace a regular meal. For example, the composition may serve as a supplemental nutritional source or the sole source of nutrition for the subject. Wherein the composition is administered as the sole source of nutrition, this may be the sole source of nutrition replacing a single meal or may be the sole source of nutrition replacing the subject's normal nutritional intake over a defined period, for example, a period of at least 8 hours, a period of at least 24 hours, a period of at least 7 days.

For embodiments wherein the composition is administered as part of a regular meal, the composition may be administered, for example consumed, prior to or during consumption of the meal. In embodiments wherein the composition is administered to replace a regular meal or is intended to serve as the sole source of nutrition for the subject, the composition may be formulated as a nutritional product intended to provide a certain amount of energy per serving. For example, the nutritional composition may be formulated so as to provide up to 500 kcal of energy per serving, including from 20 kcal to 500 kcal, from 75 kcal to 500 kcal, from 150 kcal to 500 kcal, from 200 kcal to 500 kcal, from 300 kcal to 500 kcal, or from 400 kcal to 500 kcal per serving.

### C. Compositions for administration

The methods as described herein involve administering to a subject a composition comprising a specific carbohydrate system comprising at least one slow rate of digestion simple carbohydrate, at least one complex carbohydrate, and at least one further carbohydrate selected from a nonabsorbent carbohydrate and an indigestible carbohydrate. In certain preferred embodiments, the composition is a nutritional composition.

The actual product form of the composition administered to the subject is not critical so long as the carbohydrate system is delivered to the subject at a dose effective to achieve the effects described in section B above. The product forms described herein below should be viewed as exemplary and not limiting in any manner as other product forms not listed herein are within the scope of the present disclosure.

In accordance with the methods described herein, the composition may be formulated as a nutritional composition according to any of the embodiments described below. The subject may be administered a single serving or multiple (e.g. two, three, four or more) servings per day wherein a serving is dependent on the formulation of the composition, for example as a liquid or solid.

The compositions or nutritional compositions of the present disclosure may be formulated and administered in any known or otherwise suitable oral product form. Any nutritional solid, semi-solid, liquid, semi-liquid, or powder form, including combinations or variations thereof, are suitable for use herein, provided that such forms allow for safe and effective oral delivery to the individual of the carbohydrate system as described herein. In one specific embodiment, the nutritional composition is in the form of a bar, such as a nutritional bar, weight loss bar, or meal replacement bar.

The compositions or nutritional compositions of the present disclosure may be formulated as dietary product forms, which are defined herein as those embodiments comprising the carbohydrate system as described herein in a product form that also contains at least one of fat and protein, and optionally, additional carbohydrates. The compositions may be formulated with sufficient kinds and amounts of nutrients to provide a sole, primary, or supplemental source of nutrition, or to provide a specialized nutritional product for use in subjects afflicted with specific diseases or conditions or with a targeted nutritional benefit.

### Nutritional Liquids

Nutritional liquids include both concentrated and ready-to-feed nutritional liquids. These nutritional liquids are most typically formulated as suspensions, emulsions or clear or substantially clear liquids.

Nutritional emulsions suitable for use may be aqueous emulsions comprising proteins, fats, and carbohydrates. These emulsions are generally flowable or drinkable liquids at from about 1°C to about 25°C and are typically in the form of oil-in-water, water-in-oil, or complex aqueous emulsions, although such emulsions are most typically in the form of oil-in-water emulsions having a continuous aqueous phase and a discontinuous oil phase.

The nutritional liquids may be and typically are shelf stable. The nutritional liquids typically contain up to about 95% by weight of water, including from about 50% to about 95%, also including from about 60% to about 90%, and also including from about 70% to about 85%, of water by weight of the nutritional liquid. The nutritional liquids may have a variety of product densities, but most typically have a density greater than about 1.03 g/mL, including greater than about 1.04 g/mL, including greater than about 1.055 g/mL, including from about 1.06 g/mL to about 1.12 g/mL, and also including from about 1.085 g/mL to about 1.10 g/mL.

The nutritional liquid may have a pH ranging from about 3.5 to about 8, but are most advantageously in a range of from about 4.5 to about 7.5, including from about 5.5 to about 7.3, including from about 6.2 to about 7.2.

Although the serving size for the nutritional liquid can vary depending upon a number of variables, a typical serving size is generally at least about 2 mL, or even at least about 5 mL, or even at least about 10 mL, or even at least about 25 mL, including ranges from about 2 mL to about 300 mL, including from about 100 mL to about 300 mL, from about 4 mL to about 250 mL, from about 150 mL to about 250 mL, from about 10 mL to about 240 mL, and from about 190 mL to about 240 mL.

### Nutritional Powders

The nutritional powders are in the form of flowable or substantially flowable particulate compositions, or at least particulate compositions. Particularly suitable nutritional powder forms include spray dried, agglomerated or dry-blended powder compositions, or combinations thereof, or powders prepared by other suitable methods. The compositions can easily be scooped and measured with a spoon or similar other device, wherein the compositions can easily be reconstituted with a suitable aqueous liquid, typically water, to form a nutritional liquid for immediate oral or enteral use. In this context, "immediate" use generally means within about 48 hours, most typically within about 24 hours, preferably right after or within 20 minutes of reconstitution.

### Solid Nutritional Products

The compositions for use according to the methods described herein may be formulated as a solid nutritional composition. Examples of suitable solid nutritional compositions for use herein include snack and meal replacement products, including those formulated as bars; sticks; cookies, breads, cakes, or other baked goods; frozen liquids; candy; breakfast cereals; reconstitutable powders, granulated solids, or other particulates; snack chips or bites; frozen or retorted entrees; and so forth. In certain exemplary embodiments, when the nutritional composition is formulated as a solid nutritional composition, the serving is within a range of about 25 grams to about 150 grams.

The various embodiments of the nutritional compositions of the present disclosure may be substantially free of any optional or selected ingredient or feature described herein, provided that the remaining nutritional compositions still contain all of the required ingredients or features as described herein. In this context, and unless otherwise specified, the term "substantially free" means that the selected nutritional compositions contain less than a functional amount of the optional ingredient, typically less than 1%, including less than 0.5%, including less than 0.1%, and also including zero percent, by weight of such optional or selected ingredient.

The nutritional compositions and methods of the present disclosure may comprise, consist of, or consist essentially of the essential elements of the products and methods as described herein, as well as any additional or optional element described herein or otherwise useful in nutritional compositions.

### D. The carbohydrate system

The methods of the present invention utilize a composition or nutritional composition that includes a carbohydrate system as described herein. The carbohydrate system may include all of the carbohydrate components present in the nutritional composition such that the nutritional composition does not contain any other carbohydrates components, or may include only a portion of the carbohydrate components present in the nutritional composition; that is, in some embodiments there are additional carbohydrate components present in the nutritional composition in addition to the carbohydrate system as described herein such as, for example, lactose.

The carbohydrate systems as described herein may comprise specific combinations of carbohydrates that provide a low glycemic load, generally less than about 55, or less than about 50, or less than about 40, or less than about 45, or less than about 30, or less than about 28, or about 27. As used herein, the experimental glycemic load is determined experimentally by feeding human test subjects 50 g of available CHO comprising 5.5% lactose + 8.8% maltodextrin DE 9-16 + 75% Isomaltulose + 10.7% Fibersol 2E after an overnight fast, expressed as a percentage of the response after 50 g anhydrous glucose was taken by the same subject. This procedure is described in Wolever et al, Measuring the Glycemic Index of Foods: Interlaboratory Study, Am J Clin Nutr. 2008 Jan;87(1):247S-257S, the contents of which are incorporated herein in their entirety. This is in contrast to the "daily glycemic load" or "DLG" of the experimental rodent diet, in which DGL is determined theoretically considering the amount of each individual carbohydrate contained in the CHO mixture, the glycemic index of the CHO mixture and the total dietary intake.

The carbohydrate systems of the present disclosure include at least one simple carbohydrate that has a slow rate of digestion. Simple carbohydrates include those carbohydrates that are comprised of monosaccharide sugars or disaccharide sugars. Carbohydrates that have a slow rate of digestion (or slowly digestible carbohydrate) are carbohydrates that are low glycemic and/or low insulinemic. These carbohydrates generally provide a gradual, relatively low rise in blood glucose over time. Carbohydrates that have a slow rate of digestion may be defined as "carbohydrates that are likely to be completely digested in the small intestine but at a slower rate than other carbohydrates".

Slowly digestible carbohydrates can be identified or defined using the methods described in Englyst et al., 1992 Eur J Clin Nutr 46 (Suppl.2), S33-S50 (Classification and measurement of nutritionally important starch fractions), the contents of which are incorporated herein in their entirety.

Suitable simple carbohydrates that have a slow rate of digestion that are suitable for use in the carbohydrate system include isomaltulose, sucromalt, and combinations thereof. Isomaltulose is a disaccharide (sucrose isomer) comprised of glucose and fructose linked together by 1,6 linkages; it is naturally present in honey and sugar cane. Sucromalt may be made from the enzymatic conversion of sucrose and maltose into a fructose and oligosaccharide liquid syrup. This oligosaccharide is comprised of glucoses linked together by alternating 1,3 and 1,6 linkages.

The at least one simple carbohydrate that has a slow rate of digestion may be present in the carbohydrate system in an amount of from about 40% to about 80% by weight, including from about 40% to about 75% by weight, including from about 40% to about 70% by weight, including from about 45% to about 70% by weight, including from about 50% to about 70% by weight, including from about 55% to about 70% by weight, including from about 60% to about 70% by weight, including from about 65% to about 70% by weight. In some specific embodiments, the simple carbohydrate that has a slow rate of digestion may be present in the carbohydrate system in an amount of about 65% by weight, or even about 66% by weight, or even about 67% by weight, or even about 68% by weight, or even about 69% by weight, or even about 70% by weight. In embodiments wherein there are two or more simple carbohydrates present in the carbohydrate system, the amounts above may represent the total simple carbohydrate component of the carbohydrate system.

In addition to the simple carbohydrate that has a slow rate of digestion, the carbohydrate system includes at least one complex carbohydrate. Complex carbohydrates consist of three or more single sugar molecules linked together. As used herein a "single sugar molecule" means a single monosaccharide unit. Suitable complex carbohydrates for use in the carbohydrate system include, for example, maltodextrins. In some particularly desirable embodiments, the maltodextrins will have a Dextrose Equivalent of from about 5 to about 25, or about 9 to about 16. Other suitable complex carbohydrates in some embodiments include other sources of starches such as, for example, corn starch, rice starch, wheat starch, and the like.

The at least one complex carbohydrate may be present in the carbohydrate system in an amount of from about 1% to about 15% by weight, including from about 2% to about 12% by weight, including from about 2% to about 10% by weight, including from about 3% to about 10% by weight, including from about 4% to about 10% by weight, including from about 5% to about 10% by weight, including from about 6% to about 10% by weight, including from about 7% to about 10% by weight, and including from about 8% to about 10% by weight. In some particularly desirable embodiments, the complex carbohydrate is present in the carbohydrate system in an amount of about 8% by weight, including about 9% by weight, including about 10% by weight. In embodiments wherein there are two or more complex carbohydrates present in the carbohydrate system, the amounts above may represent the total complex carbohydrate component of the carbohydrate system.

In addition to the simple carbohydrate that has a slow rate of digestion and the complex carbohydrate, the carbohydrate systems as described herein additionally include at least one of: (1) a nonabsorbent carbohydrate; and/or (2) an indigestible carbohydrate. In some embodiments of the present disclosure, the carbohydrate system will comprise, consist essentially of, or consist of a simple carbohydrate that has a slow rate of digestion, a complex carbohydrate, and a nonabsorbent carbohydrate. In other embodiments of the present disclosure, the carbohydrate system will comprise, consist essentially of, or consist of a simple carbohydrate that has a slow rate of digestion, a complex carbohydrate, and an indigestible carbohydrate. In still other embodiments of the present disclosure, the carbohydrate system will comprise, consist essentially of, or consist of a simple carbohydrate that has a slow rate of digestion, a complex carbohydrate, a nonabsorbent carbohydrate, and an indigestible carbohydrate. In some embodiments, as noted above, one or more additional carbohydrates, such as lactose, may be present in addition to the carbohydrate system.

In some embodiments, the carbohydrate system includes a nonabsorbent carbohydrate. Nonabsorbent carbohydrates include fibers and other non-absorbable starches that are not substantially absorbed in the upper intestinal tract so that they pass through to the colon where bacteria ferment them into fatty acids that can be absorbed. These fatty acids may act to heal the lining of the colon. Suitable nonabsorbent carbohydrates include inulin, and insoluble dietary fibers, including Fibersol® fibers, including Fibersol® 2E, which is a digestion-resistant maltodextrin, Nutriose® , amylose, or other insoluble fibers, and combinations thereof.

The nonabsorbent carbohydrate may be present in the carbohydrate system in an amount of from about 5% to about 25% by weight, including from about 5% to about 20% by weight, including from about 5% to about 19% by weight, including from about 5% to about 18% by weight, including from about 5% to about 17% by weight, including from about 5% to about 16% by weight, including from about 7.0% to about 16% by weight, including from about 7.0% to about 15.5% by weight. In some embodiments, the nonabsorbent carbohydrate is present in the carbohydrate system in an amount of about 7.0% by weight. In another embodiment, the nonabsorbent carbohydrate is present in the carbohydrate system in an amount of about 15.5% by weight. In embodiments wherein there are two or more nonabsorbent carbohydrates present in the carbohydrate system, the amounts above may represent the total nonabsorbent carbohydrate component of the carbohydrate system.

In some embodiments, the carbohydrate system includes an indigestible carbohydrate. Indigestible carbohydrates are carbohydrates, including some fibers, that promote digestion and a healthy bowel. Suitable indigestible carbohydrates include fructooligosaccharides, galactooligosaccharides, trans-galactooligosaccharides, xylooligosaccharides, and combinations thereof.

The indigestible carbohydrate may be present in the carbohydrate system in an amount of from about 1.0% to about 18% by weight, including from about 2% to about 17% by weight, including from about 2% to about 15% by weight, including from about 3% to about 15% by weight, including from about 3% to about 14% by weight, including from about 3% to about 13% by weight, including from about 3% to about 12% by weight. In one particularly desirable embodiment, the indigestible carbohydrate is present in the carbohydrate system in an amount of about 3.5% by weight. In another particularly desirable embodiment, the indigestible carbohydrate is present in the carbohydrate system in an amount of about 12% by weight. In embodiments wherein there are two or more indigestible carbohydrates present in the carbohydrate system, the amounts above may represent the total indigestible carbohydrate component of the carbohydrate system.

In certain embodiments, the carbohydrate system comprises or consists of a slow rate of digestion simple carbohydrate that is isomaltulose, a complex carbohydrate that is maltodextrin having a DE of 9 to 16, a nonabsorbent carbohydrate that is Fibersol 2E and an indigestible carbohydrate that is a fructooligosaccharide.

In a particularly desirable embodiment, the carbohydrate system comprises or consists of about 68% by weight isomaltulose, about 8.0% by weight maltodextrin having a DE of 9 to 16, about 12% by weight fructooligosaccharides, about 7.0 % by weight Fibersol 2E insoluble dietary fiber, and a maximum of 5.0% by weight lactose.

In another particularly desirable embodiment, the carbohydrate system comprises or consists of about 68% by weight isomaltulose, about 8.0% by weight maltodextrin having a DE of 9 to 16, about 3.5% by weight fructooligosaccharides, about 15.5 % by weight Fibersol 2E insoluble dietary fiber, and a maximum of 5.0% by weight lactose.

### E. Additional ingredients

### Macronutrients

The compositions including the carbohydrate systems as described herein may further comprise one or more optional additional macronutrients. The optional macronutrients include proteins, lipids, and carbohydrates in addition to the carbohydrate system described above, and combinations thereof. In some embodiments, the nutritional compositions are formulated as dietary products containing all three macronutrients for the subject consuming the composition.

Macronutrients suitable for use herein include any protein, lipid, or carbohydrate (in addition to the carbohydrate system) or source thereof that is known for or otherwise suitable for use in an oral nutritional composition, provided that the optional macronutrient is safe and effective for oral administration and is otherwise compatible with the other ingredients in the nutritional composition.

The concentration or amount of optional lipid, carbohydrate (including the carbohydrate system described herein), and protein in the nutritional compositions can vary considerably depending upon the particular product form (e.g., bars or other solid dosage forms, milk or soy-based liquids or other clear beverages, reconstitutable powders, etc.) and the various other formulations and targeted dietary needs. These optional macronutrients are most typically formulated within any of the embodied ranges described in Table 3.

**Table 3 Nutrient sources of exemplary nutritional compositions**

| **Nutrient % Total Cal.** | **Embodiment A** | **Embodiment B** | **Embodiment C** |
|---|---|---|---|
| **Carbohydrate** | **0-98** | **2-96** | **10-75** |
| **Protein** | **0-98** | **2-96** | **5-70** |
| **Lipid** | **0-98** | **2-96** | **20-85** |

| | **Embodiment D** | **Embodiment E** | **Embodiment F** |
|---|---|---|---|
| **Carbohydrate** | **30-50** | **25-50** | **25-50** |
| **Protein** | **15-35** | **10-30** | **5-30** |
| **Lipid** | **35-55** | **1-20** | **2-20** |

Each numerical value may be preceded by the term "about"

### Carbohydrate

Optional carbohydrates suitable for use in the nutritional compositions, in addition to the carbohydrate systems described herein, may be simple, complex, or variations or combinations thereof. Non-limiting examples of suitable carbohydrates include hydrolyzed or modified starch or cornstarch, maltodextrin, isomaltulose, sucromalt, glucose polymers, sucrose, corn syrup, corn syrup solids, rice-derived carbohydrate, glucose, fructose, lactose, high fructose corn syrup, honey, sugar alcohols (e.g., maltitol, erythritol, sorbitol), and combinations thereof.

Optional carbohydrates suitable for use herein also include soluble dietary fiber, non-limiting examples of which include gum Arabic, fructooligosaccharides (FOS), sodium carboxymethyl cellulose, guar gum, citrus pectin, low and high methoxy pectin, oat and barley glucans, carrageenan, psyllium, and combinations thereof. Insoluble dietary fiber is also suitable as a carbohydrate source herein, non-limiting examples of which include oat hull fiber, pea hull fiber, soy hull fiber, soy cotyledon fiber, sugar beet fiber, cellulose, corn bran, and combinations thereof.

### Protein

Optional proteins suitable for use in the nutritional compositions include hydrolyzed, partially hydrolyzed or non-hydrolyzed proteins or protein sources, and can be derived from any known or otherwise suitable source such as milk (e.g., casein, whey), animal (e.g., meat, fish, egg albumen), cereal (e.g., rice, corn), vegetable (e.g., soy, pea, potato), or combinations thereof. The proteins for use herein can also include, or be entirely or partially replaced by, free amino acids known for use in nutritional products, non-limiting examples of which include L-tryptophan, L-glutamine, L-tyrosine, L-methionine, L-cysteine, taurine, L-arginine, L-carnitine, and combinations thereof.

### Lipid

Optional lipids suitable for use in the nutritional composition include coconut oil, fractionated coconut oil, soy oil, corn oil, olive oil, safflower oil, high oleic safflower oil, high GLA-safflower oil, MCT oil (medium chain triglycerides), sunflower oil, high oleic sunflower oil, palm and palm kernel oils, palm olein, canola oil, flaxseed oil, borage oil, cottonseed oils, evening primrose oil, blackcurrant seed oil, transgenic oil sources, fungal oils, algae oils, marine oils (e.g., tuna, sardine), and so forth.

### Other optional ingredients

The compositions or nutritional compositions as described herein may further comprise other optional ingredients that may modify the physical, chemical, aesthetic or processing characteristics of the products or serve as pharmaceutical or additional nutritional components when used in the targeted population. Many such optional ingredients are known or otherwise suitable for use in medical food or other nutritional products or pharmaceutical dosage forms and may also be used in the compositions herein, provided that such optional ingredients are safe for oral administration and are compatible with the essential and other ingredients in the selected product form.

Non-limiting examples of such optional ingredients include preservatives, anti-oxidants, emulsifying agents, buffers, human milk oligosaccharides and other prebiotics, probiotics, nucleotides, carotenoids, pharmaceutical actives, additional nutrients as described herein, colorants, flavors, thickening agents and stabilizers, emulsifying agents, lubricants, and so forth, and combinations thereof.

A flowing agent or anti-caking agent may be included in the nutritional compositions as described herein to retard clumping or caking of the powder over time and to make a powder embodiment flow easily from its container. Any known flowing or anti-caking agents that are known or otherwise suitable for use in a nutritional powder or product form are suitable for use herein, non-limiting examples of which include tricalcium phosphate, silicates, and combinations thereof. The concentration of the flowing agent or anti-caking agent in the nutritional product varies depending upon the product form, the other selected ingredients, the desired flow properties, and so forth, but most typically range from about 0.1 % to about 4%, including from about 0.5% to about 2%, by weight of the composition.

A stabilizer may also be included in the nutritional compositions. Any stabilizer that is known or otherwise suitable for use in a nutritional product is also suitable for use herein, some non-limiting examples of which include gums such as xanthan gum. The stabilizer may represent from about 0.1 % to about 5.0%, including from about 0.5% to about 3%, including from about 0.7% to about 1.5%, by weight of the nutritional composition.

The nutritional composition may further comprise any of a variety of vitamins, non-limiting examples of which include vitamin A, vitamin D, vitamin E, vitamin K, thiamine, riboflavin, pyridoxine, vitamin B12, niacin, folic acid, pantothenic acid, biotin, vitamin C, choline, inositol, salts and derivatives thereof, and combinations thereof.

The nutritional composition may also further comprise any of a variety of minerals known or otherwise suitable for use in nutritional compositions, non-limiting examples of which include phosphorus, magnesium, calcium as described hereinbefore, zinc, manganese, copper, iodine, sodium, potassium, chloride, selenium, and combinations thereof.

### F Methods of Manufacture

The compositions or nutritional compositions for administration according to the methods of the present invention may be prepared by any known or otherwise effective manufacturing technique for preparing the selected product solid or liquid form. Many such techniques are known for any given product form such as nutritional liquids or powders and can easily be applied by one of ordinary skill in the art to the compositions described herein.

The compositions can therefore be prepared by any of a variety of known or otherwise effective formulation or manufacturing methods. In one suitable manufacturing process, for example, at least two separate slurries are prepared, that are later blended together, heat treated, standardized, and either terminally sterilized to form a retort composition or aseptically processed and filled to form an aseptic composition. Alternately, the slurries can be blended together, heat treated, standardized, heat treated a second time, evaporated to remove water, and spray dried to form a powder composition.

The slurries formed may include a carbohydrate-mineral (CHO-MIN) slurry and a protein-in-fat (PIF) slurry. Initially, the CHO-MIN slurry is formed by dissolving selected carbohydrates (e.g., carbohydrate system, etc.) in heated water with agitation, followed by the addition of minerals (e.g., potassium citrate, magnesium chloride, potassium chloride, sodium chloride, choline chloride, etc.). The resulting CHO-MIN slurry is held with continued heat and moderate agitation until it is later blended with the other prepared slurries.

The PIF slurry may be formed by heating and mixing the oil (e.g., high oleic safflower oil, soybean oil, coconut oil, monoglycerides, etc.) and emulsifier (e.g., soy lecithin), and then adding oil soluble vitamins, mixed carotenoids, protein (e.g., whey protein, casein protein, etc.), carrageenan (if any), calcium carbonate or tricalcium phosphate (if any), and ARA oil and DHA oil (in some embodiments) with continued heat and agitation. The resulting PIF slurry is held with continued heat and moderate agitation until it is later blended with the other prepared slurries.

Water is heated and then combined with the CHO-MIN slurry, nonfat milk (if any), and the PIF slurry under adequate agitation. The pH of the resulting blend is adjusted to 6.6-7.0, and the blend is held under moderate heated agitation. ARA oil and DHA oil is added at this stage in some embodiments.

The composition is then subjected to high-temperature short-time (HTST) processing, during which the composition is heat treated, emulsified and homogenized, and then cooled. Water soluble vitamins and ascorbic acid are added, the pH is adjusted to the desired range if necessary, flavors (if any) are added, and water is added to achieve the desired total solid level. For aseptic compositions, the emulsion receives a second heat treatment through an aseptic processor, is cooled, and then aseptically packaged into suitable containers. For retort compositions, the emulsion is packaged into suitable containers and terminally sterilized. In some embodiments, the emulsions can be optionally further diluted, heat-treated, and packaged to form a desired ready-to-feed or concentrated liquid, or can be heat-treated and subsequently processed and packaged as a reconstitutable powder, e.g., spray dried, dry mixed, agglomerated.

The spray dried composition or dry-mixed composition may be prepared by any collection of known or otherwise effective techniques, suitable for making and formulating a nutritional powder. For example, when the powder composition is a spray-dried nutritional powder, the spray drying step may likewise include any spray drying technique that is known for or otherwise suitable for use in the production of nutritional powders. Many different spray drying methods and techniques are known for use in the nutrition field, all of which are suitable for use in the manufacture of the spray dried powder composition. Following drying, the finished powder may be packaged into suitable containers. Dry-blending or dry-mixing may also be used to prepare the compositions of the present disclosure.

The invention will now be understood with reference to the following non-limiting examples.

### EXAMPLES

### Example 1

A study was carried out to investigate the effect of a nutritional composition comprising a specific carbohydrate system in a well-established rat model of obesity. The study aimed to evaluate the effect of the carbohydrate system on satiety and energy expenditure as a nutritional strategy to prevent obesity.

*Experimental design -* Protocols for all experimental procedures were conducted in accordance with the ethical guidelines for animal experimentation at the Spanish National Research Council (RD1201/2005 October 10). Three-week old male Wistar rats (n=30) were purchased from Harlan (Barcelona, Spain) and housed in cages in a temperature-controlled room (22±2°C) with a 12h light-dark cycle. Water and chow diet were available *ad libitum* until starting the experiment.

Rats were fed a high fat (HF) diet for 12 weeks to induce obesity. The HF diet contained 20% fat, 24% protein, 42% carbohydrates and 8% fiber approx. Rats were then randomly assigned to one of three dietary experimental groups: HF group - rats remained feeding with HF/HC diet *ad libitum;* LC group - rats were fed a HF diet containing carbohydrates with a low digestion rate *ad libitum;* PF group - rats received the same daily amount of HF/HC diet as the LC group based on the LC group's daily intake. This last group is necessary to determine whether the possible effects of the diet on weight loss are due to either a reduction in food intake or could be induced by additional mechanisms involved in the regulation of satiety control. All diets are listed in Table 4. Animals were fed these diets for 8 weeks.

**Table 4**

| **Experimental groups** | | **LC** | **HF** | **PF** |
|---|---|---|---|---|
| **Experimental rodent diets** | | **HF/LC** | **HF/HC** | |
| **Total protein** | | **24.19** | **24.19** | |
| | **Calcium caseinate** | **97.65** | **97.81** | |
| **Total Fat** | | **20.50** | **20.50** | |
| | **Lard fat** | **98.46** | **99.99** | |
| **Total HC** | | **49.11** | **49.42** | |
| | **Maltodextrin (GI=95)** | **7.76** | **24.43** | |
| | **Lactose (GI=46)** | **5.15** | **-** | |
| | **Isomaltulose (GI=32)** | **67.88** | **-** | |
| | **Sucrose (GI=65)** | **0.33** | **36.07** | |
| | **Glucose (GI=100)** | **0.08** | **-** | |
| | **Cornstarch (GI=85)** | **-** | **31.60** | |
| | **Fibersol 2E (GI=5)** | **15.47** | **-** | |
| | **FOS (GI=0)** | **3.49** | **-** | |
| | **Cellulose (GI=0)** | **-** | **7.90** | |

Body weight and food intake were recorded every 3-4 days before the onset of the dark period. Analyses of body compositions were performed using nuclear magnetic resonance (NMR, EchoMRI, EchoMedical Systems, Houston TX) at the 90^{th} day of the experimental dietary period.

After around 60 days of feeding with the different experimental diets, determinations of indirect calorimetry were performed in an open circuit calorimetry system (Oxylet, Panlab, Barcelona, Spain). Briefly, rats were individually housed for 24 h in airtight metabolic chambers under the same light and temperature conditions as described above. Data relating to oxygen consumption and carbon dioxide production were used to calculate the respiratory quotient (RQ) and the energy expenditure (EE) for each animal.

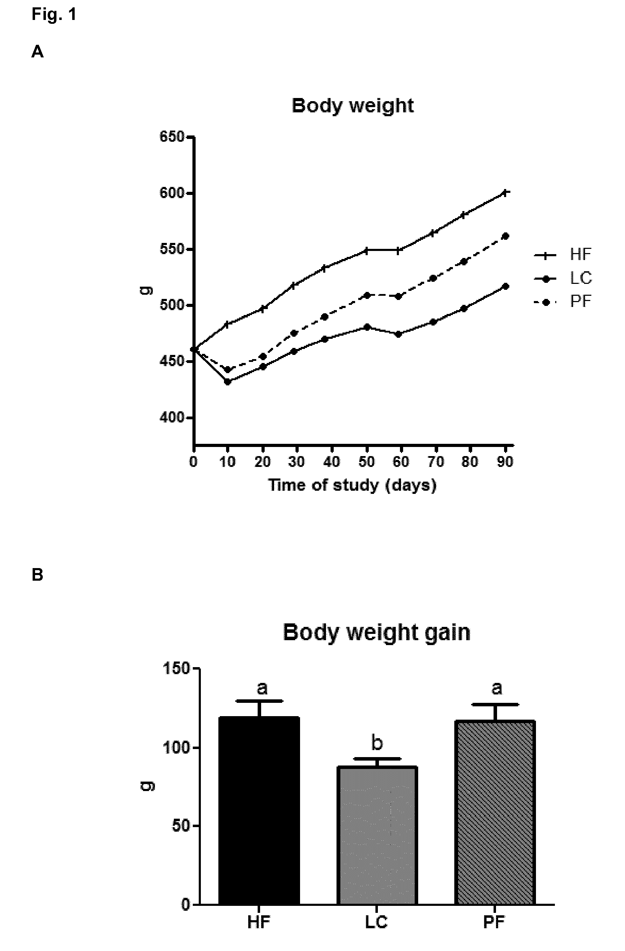

*Results -* As shown in Fig. 1A, initial body weight was similar among the three groups. Rats from the HF group had a body weight gain significantly higher than the LC group over the duration of the experimental period. Body weight evolution of rats from the PF group was significantly higher than that of rats from group LC and lower than that of rats from the HF group.

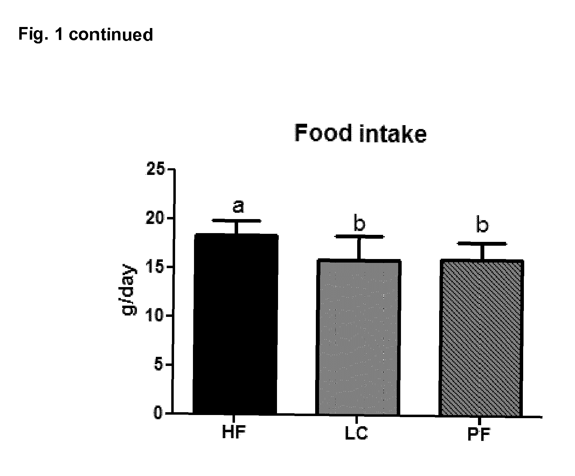

At the end of the experiment (Fig. 1B), the rodent diet containing slowly absorbed carbohydrates (HF/LC diet) induced lower body weight gain in comparison to the HF/HC diet regardless of the amount consumed by the animals. Thus the PF group had a greater body weight gain than the LC group even though they consumed the same amount of diet (Fig. 1C). Therefore, the effects of the HF/LC diet may not only be attributed to a reduced food intake.

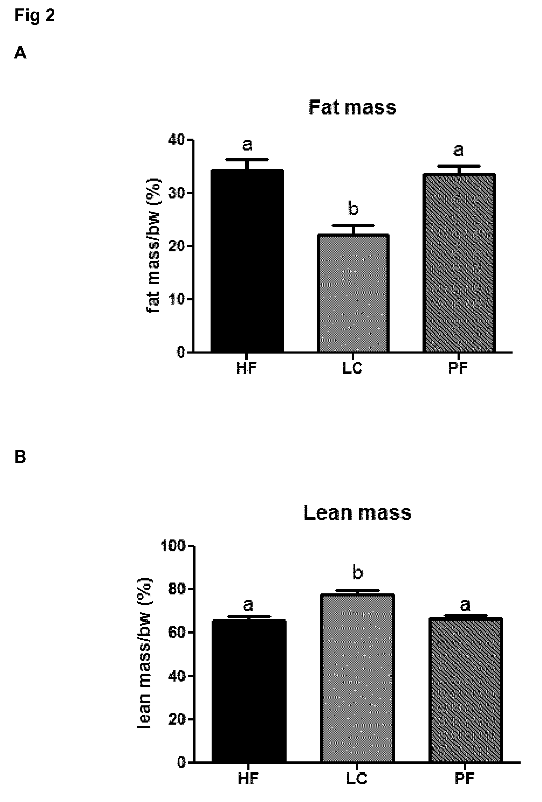

Body composition analyses were performed after 90 days of feeding the study diets. The analysis of body composition at the end of the experimental period revealed that the diet with slowly digesting carbohydrates significantly reduced fat mass content as compared to both HF and PF groups (Fig. 2A). Interestingly, the reduction in body weight gain observed in the LC group was accompanied by a significant increase in lean mass percentage (p<0.05) when compared with the HF and PF groups (Fig. 2B).

All these data demonstrate that a diet supplemented with slow digesting carbohydrates is able to reduce body weight gain in an animal obesity model induced by a HF/HC diet. However, taking into account that the animals from the PF group consumed the same amount of food as the LC animals, and that the energy content of the different experimental diets is similar, it was hypothesized that the decrease observed in the body weight gain in the LC group could not only be due to a reduction in energy intake, but also could be associated with other mechanisms involved in the control of energy balance.

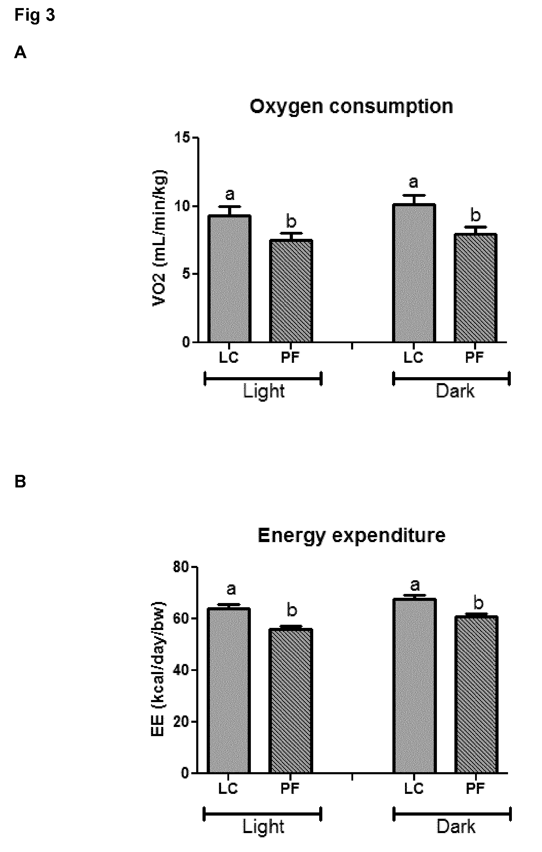

To further evaluate the impact of this specific carbohydrate system on whole body energy expenditure (EE) in the LC and PF groups, total daily caloric expenditure was monitored by using indirect calorimetry. As shown in Fig. 3A and 3B respectively, the LC group significantly increased the diurnal and nocturnal (p<0.05) oxygen consumption (VO2) and also the overall energy expenditure (EE) as compared to the PF group. Taking into account that the energy food intake was similar between both groups, these results suggest that consumption of slowly absorbed carbohydrates are able to modulate energy balance by enhancing oxygen consumption and, therefore, the EE, which may also contribute to the reduction in body weight gain observed in the LC group compared to its pair-fed group.

In summary, the diet with the specific carbohydrate blend reduced the body weight gain at least by two different mechanisms involving satiety control as well as energy expenditure.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. Moreover, all aspects and embodiments of the invention described herein are considered to be broadly applicable and combinable with any and all other consistent embodiments, including those taken from other aspects of the invention (including in isolation) as appropriate.

Various publications and patent applications are cited herein, the disclosures of which are incorporated by reference in their entireties.

## Claims

1. A method of inducing satiety in a subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate.

2. The method of claim 1 wherein satiety is induced such that the subject's energy intake is reduced.

3. A method of increasing energy expenditure in subject, the method comprising administering to the subject a composition comprising at least one slow rate of digestion simple carbohydrate, at least one complex carbohydrate, and at least one further carbohydrate selected from a nonabsorbent carbohydrate and an indigestible carbohydrate.

4. A composition comprising at least one slow rate of digestion simple carbohydrate, at least one complex carbohydrate, and at least one further carbohydrate selected from a nonabsorbent carbohydrate and an indigestible carbohydrate for use in preventing or treating obesity in a subject.

5. The composition for use according to claim 4, wherein the composition administered to the subject induces satiety and/or increases energy expenditure in the subject.

6. The method of any of claims 1-3 or composition for use according to claim 4 or claim 5 wherein the subject is a human or wherein the subject is selected from a child, a toddler, an adolescent, an adult or an elderly adult.

7. The method or composition for use according to claim 6 wherein the subject is not a pregnant woman or a lactating woman.

8. The method or composition for use according to any of claims 1-7 wherein the composition comprises at least one slow rate of digestion simple carbohydrate, at least one complex carbohydrate, at least one nonabsorbent carbohydrate and at least one indigestible carbohydrate.

9. The method or composition for use according to any of claims 1-8 wherein the slow rate of digestion simple carbohydrate is a carbohydrate comprised of monosaccharide or disaccharide sugars and that is low glycemic and/or low insulinemic such that it provides a relatively low rise in blood glucose over time and/or wherein the slow rate of digestion simple carbohydrate is selected from the group consisting of isomaltulose and sucromalt.

10. The method or composition for use according to any of claims 1-9 wherein the complex carbohydrate is a carbohydrate comprising a chain of three or more monosaccharides and/or wherein the complex carbohydrate is selected from the group consisting of a maltodextrin, corn starch, rice starch and wheat starch.

11. The method or composition for use according to any of claims 1-10 wherein the complex carbohydrate comprises maltodextrin having a Dextrose Equivalent of from 3 to 25 or comprises maltodextrin having a Dextrose Equivalent of from 9 to 16.

12. The method or composition for use according to any of claims 1-11 wherein the nonabsorbent carbohydrate is a carbohydrate that is not absorbed in the upper intestinal tract or wherein the nonabsorbent carbohydrate is selected from the group consisting of inulin, dietary insoluble fibers, and digestion-resistant maltodextrins, and/or wherein the indigestible carbohydrate is selected from the group consisting of fructooligosaccharides, galactooligosaccharides, trans-galactooligosaccharides and xylooligosaccharides.

13. The method or composition for use according to any of claims 1-12 wherein the composition comprises a slow rate of digestion simple carbohydrate that is isomaltulose, a complex carbohydrate that is maltodextrin having a DE of 9 to 16, a nonabsorbent carbohydrate that is Fibersol 2E and an indigestible carbohydrate that is fructooligosaccharide.

14. The method or composition for use according to any of claims 1-13 wherein the composition comprises from 40% to 80% by weight slow rate of digestion simple carbohydrate, from 1% to 15% by weight complex carbohydrate, from about 5% to about 25% by weight nonabsorbent carbohydrate, and from 1% to 20% by weight indigestible carbohydrate.

15. The method or composition for use according to any of claims 1-14 wherein the composition is a nutritional composition, optionally wherein the nutritional composition comprises one or more additional macronutrients, optionally selected from protein, lipid and/or additional carbohydrate.
